# EUROPÄISCHE PATENTSCHRIFT

(11) **EP 0 968 993 B1**
(45) Veröffentlichungstag und Bekanntmachung des Hinweises auf die Patenterteilung: **05.06.2002**
(21) Anmeldenummer: 99112240.9
(22) Anmeldetag: 25.06.1999
(51) Int. Cl.: C07C 51/487, C11C 1/10, C07C 53/126, C07C 57/03, C07C 57/12, C07C 67/08

(54) **Verfahren zur Behandlung natürlicher Fettsäuren**
Process for the treatment of natural fatty acids
Procédé pour le traitement d'acides gras naturels

(30) Priorität: 30.06.1998 DE 19829064; 29.05.1999 DE 19924760
(43) Veröffentlichungstag der Anmeldung: 05.01.2000
(73) Patentinhaber: DHW Deutsche Hydrierwerke GmbH Rodleben, 06862 Rodleben (DE)
(72) Erfinder: Wieczorek, Frank, Dr.rer.nat.Dipl.-Chem., 40670 Meerbusch (DE); Konetzke, Gerhard, Dr.rer.nat.Dipl.-Chem., 06847 Dessau (DE); Schmidt, Klaus, Dr.rer.nat.Dipl.-Chem., 06862 Rodleben (DE); Weidemann, Reinhold, Dipl.-Ing., 14827 Wiesenburg (DE); Bär, Wolfgang, Dipl.-Ing.(FH), 06862 Rodleben (DE)
(74) Vertreter: Tragsdorf, Bodo, Dipl.-Ing.

(56) Entgegenhaltungen:
- EP-A- 0 334 154
- EP-A- 0 494 527
- DE-A- 1 961 861
- DE-A- 4 304 468
- DE-C- 528 361
- GB-A- 226 818

## Beschreibung

Die Erfindung betrifft ein Verfahren zur Behandlung natürlicher Fettsäuren.

Natürliche Fettsäuren stellen im wesentlichen Gemische von Monocarbonsäuren mit geradzahligen und unverzweigten C-Ketten dar, deren Sättigungsgrad jedoch unterschiedlich ist. Natürliche Fettsäuren werden aus natürlich vorkommenden Ölen und Fetten gewonnen. Je nach Herkunft der Öle und Fette und deren weiterer Verarbeitung können die daraus hergestellten Fettsäuren eine Vielzahl von Begleitstoffen in unterschiedlichen, meist jedoch äußerst kleinen Mengen enthalten. Dabei handelt es sich sowohl um unverseifbare Bestandteile als auch um bi- und polyfunktionelle Carbonsäuren, die untereinander sowie mit den Doppelbindungen und Carbonylgruppen der Fettsäuren Reaktionsprodukte bilden. Dadurch werden unerwünschte Verfärbungen der Fettsäuren verursacht, insbesondere bei deren thermischer Belastung. Die zur Charakterisierung natürlicher Fettsäuren in der Industrie üblichen Analysemethoden (fettchemische Kennzahlen, Gaschromatogramme) reichen nicht aus, um die für die Verfärbungen verantwortlichen Stoffe hinreichend zu erfassen. Deshalb werden zur Qualitätsbestimmung häufig Thermotests mit Fettsäuren oder Fettsäurederivaten durchgeführt, wobei die Testapparatur, die Expositionszeit der Probe sowie die Temperatur jeweils definiert sind.
Aus den destillativ aufbereiteten Fettsäuren werden u.a. Fettsäureester hergestellt, die z.B. zur Herstellung von Lebensmitteln, Schmierstoffen, kosmetischen und pharmazeutischen Zubereitungen sowie als Hilfsmittel in der Kunststoffindustrie eingesetzt werden. Für die genannten Verwendungszwecke werden helle und farbstabile Produkte verlangt. Da bereits Veresterungsreaktionen je nach angewendetem Verfahren hohe Temperaturen (200 ± 30°, ausgenommen enzymatische Synthese) sowie Umsetzungszeiten von einigen Stunden erfordern, ist die Bereitstellung heller und farbstabiler Fettsäuren notwendig.
Die Weiterentwicklung der Destillationstechnik für Fettsäuren (Entgasung, Entwässerung, Vorlaufabtrennung, verbesserte Vakuumerzeugung, Einsatz hochleistungsfähiger Kolonnenpackungen bei der Fraktionierung) hat dazu geführt, daß einerseits die Destillationsbedingungen der thermischen Empfindlichkeit der Produkte Rechnung tragen und andererseits Farbe und Farbstabilität beeinträchtigende Nebenbestandteile teilweise abgetrennt werden können (Fette, Seifen, Anstrichm. (1985), 87 (5), S. 208 bis 214; Fett/Lipid (1996), 98 (12), S. 402 bis 408).
Naturgemäß können durch destillative Verfahren nur solche Begleitstoffe abgetrennt werden, deren Siedepunkt ausreichend weit von denen der Zielfraktionen entfernt liegt.
Bekannt ist auch, durch chemische Umsetzungen störende Bestandteile der Rohfettsäuren zu entfernen. Als Zusatzstoffe werden z.B. aromatische und aliphatische Amine, Phenolderivate, Zinkdimethylthiocarbamat, Sulfonsäuren, Titanpulver, Thiophosphorsäurederivate, tertiäres Dibutylperoxid oder Zinnpulver eingesetzt, die in der Regel den Rohfettsäuren vor deren Destillation zugemischt werden. Der Nachteil dieser Verfahrensweise besteht vor allem darin, daß die zugesetzten Chemikalien einerseits ein erhebliches Sicherheitsrisiko darstellen und andererseits, ausgehend vom Verwendungszweck der Fettsäuren, aus diesen wieder entfernt werden müssen. Der dazu erforderliche Aufwand beeinträchtigt die Wirtschaftlichkeit dieser Verfahrensweise erheblich.
Teilweise wird bei den vorgenannten Verfahren eine Hydrierung In Gegenwart der aufgeführten Chemikalien vorgeschlagen. Die hydrierende Bleichung von Ölen und Fetten, auch als touch- oder ultralight hydrogenation bezeichnet, wird jedoch meistens ohne weitere Chemikalienzusätze nur in Gegenwart eines Hydrierkatalysators durchgeführt. Bei Hydrierversuchen zur weiteren Absenkung der Jodzahlen von gesättigten Fettsäuren konnte jedoch keine eindeutige Korrelation zwischen restlicher Jodzahl und Farbe/Thermostabilität der destillierten Fettsäuren festgestellt werden. Andererseits ist jedoch bekannt, daß durch reduktive Bedingungen, z.B. durch Behandlung mit Natriumborhydrid, tendenzielle Farbverbesserungen erzielt werden können. So wird z.B. gemäß GB-A-2072 656 die Gardner-Farbzahl = 7 von destillierter Talgölfettsäure durch NaBH₄-Behandlung des Rohproduktes auf Gardner-Farbzahl = 5 des Destillates verringert. Abgesehen davon, daß dieser Effekt für viele Anwendungsfälle nicht ausreichend ist, bewirkt die Zugabe von NaBH₄ durch die Bildung von Natriumseifen eine entsprechend größere Menge Destillationsrückstand. Die Schwierigkeiten bei der Dosierung von festem Natriumborhydrid können durch Verwendung von ebenfalls handelsüblicher NaBH₄-Lösung umgangen werden. Zur Stabilisierung enthält diese Lösung 40 % NaOH, wodurch die Seifenbildung, als Folge davon auch der Destillationsrückstand, immens vergrößert und die Wirtschaftlichkeit dieses Verfahrens verringert werden.
Gegenüber der reduktiven Bleichung, deren Effekt reversibel ist (z.B. durch Luftsauerstoff), haben oxidative Bleichungen den Vorteil, die für Produktverfärbungen verantwortlichen konjugierten C = C-Doppelbindungen irreversibel zu zerstören. Dabei werden niedermolekulare Produkte mit größerer Wasserlöslichkeit gebildet, die ausgewaschen werden können (Ullmann, 5. Edition, Vol. A 4, 191 ff. [1985]).
Geeignete Verfahren sind z.B. In den Druckschriften JP-A-48 01 8213 und
JP-A-48 01 8214 beschrieben. Demnach werden synthetische Fettsäuren (C₄ - C₂₅), deren Nebenprodukte hinsichtlich Art und Menge allerdings nicht vergleichbar sind mit denen natürlicher Festsäuren, bzw. die Na-Seifen der synthetischen Fettsäuren in wäßriger Lösung bei 65 bis 100 °C mit Wasserstoffperoxid gebleicht und anschließend nach Ansäuern mit Salzsäure gewaschen. Der Nachteil dieser Verfahren besteht hauptsächlich in einer äußerst hohen Abwasserbelastung. Der Abwasseranfall kann verringert werden, wenn anstelle der Natriumseifen die Fettsäuren direkt mit Wasserstoffperoxid behandelt werden. Der Blelcheffekt ist im sauren Bereich jedoch geringer als im alkalischen (Ullmann, 5. Edition, Vol. A 4, 194 [1985]).
Aus der JP 4-338357 A2 ist ein Verfahren zur Reinigung von Essigsäure bekannt, die aus Methanol und Kohlenmonoxid in Gegenwart eines Katalysatorsystems von Rhodium und Jodverbindungen hergestellt wurde. Die wichtigsten unerwünschten Nebenprodukte sind organische und anorganische Jodverbindungen, Form- und Acetaldehyd sowie vor allem Ameisensäure, die reduzierend wirken. Der Essigsäure werden bis zu 100 ppm Wasserstoffperoxid zugesetzt. Anschließend erfolgt eine fraktionierte Destillation unter vermindertem Druck, wodurch Wasserstoffperoxid bei < 100 °C abreagiert. Dabei entstehen Kohlendioxid und flüssige niedermolekulare Oxidationsprodukte, die als Vorlauf destillativ entfernt werden.
Diese Verfahrensweise ist zur Behandlung von natürlichen Fettsäuren jedoch ungeeignet, da natürliche Fettsäuren (mit ≥ 6 C-Atomen) deutlich andere Eigenschaften als Essigsäure aufweisen. Natürliche Fettsäuren stellen eine Mischung mehrerer Fettsäuren unterschiedlicher Kettenlänge und mit unterschiedlichem Sättigungsgrad des Alkylrestes dar und weisen deshalb ein breites Siedepunktspektrum auf, das die Siedepunkte oxidierter Derivate weitgehend einschließen würde und dadurch deren destillative Abtrennung praktisch unmöglich ist. Außerdem ist mit derart geringen Einsatzmengen an Wasserstoffperoxid eine erfolgversprechende Behandlung von natürlichen Fettsäuren zur Abtrennung unerwünschter Begleitstoffe, die eine Verfärbung der natürlichen Fettsäuren, insbesondere bei thermischer Belastung, z.B. bei Veresterungsreaktionen, bewirken, nicht möglich.
Aus der GB-226 818 A ist ein Verfahren zum Bleichen von Ölen, Fetten und Fettsäuren unter Verwendung eines Bleichmittels in wäßriger Lösung bekannt, wobei die wäßrige Lösung mehr als 15 Gew.-% H₂O₂ enthält. Gemäß dem angegebenen Beispiel 2 wird Kokosölfettsäure auf eine Temperatur von 80 °C erhitzt und unter Rühren mit einer 40%igen Wasserstoffperoxidlösung während einer Zeitdauer von einer Stunde umgesetzt. Die eingesetzte Menge an Wasserstoffperoxid beträgt 2 % bezogen auf das Gesamtgewicht der behandelten Fettsäure. Der Nachteil dieser Verfahrensweise besteht darin, daß die Reaktionsprodukte, insbesondere die in den Fettsäuren enthaltenen Komponenten mit chromophoren und auxochromen Gruppen, in der Fettsäure verbleiben und sich ungünstig auf die anwendungstechnischen Eigenschaften der Fettsäuren auswirken (Anstieg der Peroxidzahl, verringerte Thermostabilität, Ranziditätserscheinungen bei der Lagerung).

Der Erfindung lag die Aufgabe zugrunde, ein Verfahren zur Behandlung von natürlichen Fettsäuren vor deren anschließender Destillation zu schaffen, das eine wirtschaftliche, umweltschonende und gefahrlose Arbeitsweise sowie die Herstellung heller und auch nach thermischer Belastung farbstabiler Fettsäuren ermöglicht.

Erfindungsgemäß wird die Aufgabe durch die im Anspruch 1 angegebenen Merkmale gelöst. Geeignete Ausgestaltungen der Verfahrensweise sind Gegenstand der Ansprüche 2 bis 14.

Eine der wesentlichen Bedingungen der vorgeschlagenen Verfahrensweise ist die Aufheizung der Rohfettsäure auf eine Temperatur von 150 bis 230 °C, vorzugsweise auf 170 bis 210 °C, in die eine definierte Menge Wasserstoffperoxid eingetragen wird. Die Durchmischung erfolgt mittels Rührwerk oder statischem Mischer. Im Anschluß an die Wasserstoffperoxideinleitung findet während einer Zeitdauer von mindestens 10 min bei gleich hoher Temperatur die Nachreaktion statt. Bei dieser relativ hohen Temperatur werden aus dem größten Teil der in den Fettsäuren enthaltenen Chromophore, Wasserstoffperoxid und einer sehr geringen Fettsäuremenge die höhermolekularen und im späteren Destillationsrückstand verbleibenden Stoffe, insbesondere durch Kondensationsreaktionen, gebildet sowie restliche Peroxide zersetzt. Die Durchführung der Reaktion bei so hoher Temperatur hat außerdem den sicherheitstechnischen Vorteil, daß die unter Verkettung unvorhergesehener Umstände theoretisch mögliche Bildung explosionsfähiger Dampfphasen mit mehr als 26 Mol.-% H₂O₂ oberhalb 150 °C nicht möglich ist (Ullmann, 5. Edition, Vol. A 13, 462 [1989]).

Überraschenderweise zeigte sich, daß bei der Behandlung von natürlichen Fettsäuren mit Wasserstoffperoxid, jedoch nicht unter Bleichbedingungen (schwach bis stark alkalisch, T ≤ 100 °C, Auswaschung der Spaltprodukte), sondern bei wesentlich höheren Temperaturen, und ohne alkalische Zusätze, aus den in den natürlichen Fettsäuren enthaltenen Chromophoren hähermolekulare Stoffe entstehen, die bei der anschließenden und ohnehin notwendigen Destillation im Destillationsrückstand verbleiben.
Sowohl die auf diese Welse gewonnenen Fettsäuren als auch die daraus hergestellten Ester zeichnen sich durch eine hellere Farbe und verbesserte Farbstabilität gegenüber den aus gleichen Rohstoffen und nach gleichen Verfahren, jedoch ohne die beschriebene Vorbehandlung der Fettsäuren mit Wasserstoffperoxid erhaltenen Produkte aus.

Die erfindungsgemäß behandelten Fettsäuren überschreiten nicht die folgenden Farbzahlwerte nach festgelegtem Thermotest (200 °C/2h):

| | Lovibond 5 1/4" Total 10xR+G¹⁾ | APHA |
|---|---|---|
| gesättigte Fettsäuren | max. 10 | max. 100 |
| ungesättigte Fettsäuren (Oleine) | max. 30 | max. 350 |

| | | |
|---|---|---|
| ¹⁾ Zur besseren Vergleichbarkeit werden häufig die Einzelwerte Rot und Gelb der Lovibond-Farbskala zur der Größe 10xR+G zusammengefaßt. | | |

Eine zusätzliche Sicherheitsvorkehrung stellt bei Verwendung von Rührgefäßen als Reaktionsapparate die über ein Tauchrohr vorzunehmende Begasung mit Reinststickstoff (2 l/h x kg Rohfettsäure) dar. Diese Maßnahme wird in erster Linie jedoch deshalb getroffen, um das aus der Wasserstoffperoxidlösung stammende Wasser aus der Fettsäure weitestgehend zu entfernen. Die aus dem Rührgefäß abziehenden Dämpfe werden gekühlt und das Brüdenkondensat in einer Destillationsvorlage mit nicht absperrbarer Entlüftung gesammelt.
Die Stickstoffbegasung ist zweckmäßig, jedoch nicht Bedingung.
Vorzugsweise wird zur Behandlung der erhitzten Rohfettsäure eine handelsübliche 30%ige Wasserstoffperoxidlösung eingesetzt. Die Verwendung von Lösungen höherer oder geringerer Konzentration ist prinzipiell möglich. Bei geringeren H₂O₂-Konzentrationen ist jedoch die abzuführende Wassermenge zwangsläufig größer, während der Umgang mit H₂O₂-Lösungen > 30 % zusätzliche Sicherheitsvorkehrungen erfordert (Solvay Interox, Wasserstoffperoxid-Sicherheit bei Umgang und Lagerung, Techn. Inf. FP 1.2.1.).

Die Menge der einzusetzenden 30%igen Wasserstoffperoxidlösung richtet sich nach der Menge entfernbarer Chromophore aus der Rohfettsäure. Anhaltspunkte dafür sind die Farbzahlwerte und die Summe der Mengen der im Gaschromatogramm sichtbaren Nlcht-Fettsäure-Komponenten sowie die Jodzahl (JZ) bei Fettsäuren bis Kettenlänge max. C₁₂. Eine eindeutige Korrelation zwischen einem oder mehrerer Analysenwerte und der optimalen H₂O₂-Menge konnte jedoch nicht festgestellt werden. Bei erstmalig zu verarbeitenden Rohstoffpartien ist es sinnvoll, die Analysenwerte durch Ergebnisse eines unter vergleichbaren Bedingungen durchgeführten Laborversuches zu unterstützen. In den meisten Fällen sind Wasserstoffperoxidmengen (30%ig) von 1 bis 3 %, bezogen auf die Fettsäuremenge, ausreichend. Bei mehr als der unbedingt notwendigen H₂O₂-Einsatzmenge wird der Destillationsrückstand unnötigerweise vergrößert, während bei zu kleiner H₂O₂-Menge ein unterhalb der Möglichkeiten bleibender Effekt hinsichtlich Farbe und Thermostabilität erzielt wird. Wichtig bei der Festlegung der H₂O₂-Menge ist außerdem die Kenntnis über die Herkunft des Rohmaterials. Während zur Behandlung der C₆-C₁₀-Fettsäurefraktionen aus Palmkernöl 1 % H₂O₂ (30%ig) ausreicht, sind für C₆-C₁₀-Fraktionen aus Kokosölspaltfettsäure meist 3 % H₂O₂ (30%ig) und bei Anteilen der gleichen und aus der destillativen Entsäuerung von Kokosöl stammenden Fraktion bis zu 5 % H₂O₂ (30%ig) notwendig. Bei Oleinen beträgt die optimale Menge 30%iger H₂O₂-Lösung max. 1 % und bei Stearinen aus gehärteter Talgfettsäure 0,5 bis 1 %.
Hinsichtlich der Dosierungsgeschwindigkeit von Wasserstoffperoxid und der erforderlichen Nachreaktionszeit ist zwischen Chargen- und kontinuierlichem Betrieb zu unterscheiden. Bei zu rascher Dosierung würde sich der Wirkungsgrad der H₂O₂-Behandlung durch teilweise Zersetzung des Wasserstoffperoxids vor der beabsichtigten Reaktion mit den Chromophoren verringern, während bei nicht ausreichender Nachreaktionszeit organische Peroxide in der Rohfettsäure verbleiben und damit zumindest theoretisch ein Gefahrenpotential bei der nachfolgenden Destillation darstellen. Die erforderliche Nachreaktionszeit ist abhängig von der verwendeten Wasserstoffperoxidmenge und der Reaktionstemperatur.
Bei diskontinuierlicher Betriebsweise wird in einem Rührbehälter die Fettsäure vorgelegt und über eine Tauchleitung Wasserstoffperoxid dosiert. Bei einer Reaktionstemperatur von 170 °C wurde als hinreichend langsame Dosiergeschwindigkeit 0,1 bis 0,3 %/Minute, vorzugsweise 0,2 % H₂O₂ (30%ig)/Minute, und eine Nachreaktionszeit von 10 bis 30 Minuten in Abhängigkeit von der dosierten Wasserstoffperoxidmenge ermittelt. Wird die Reaktionstemperatur bei ca. 205 °C eingestellt, können die Nachreaktionszelten (bis zum vollständigen Abbau der Peroxide) ungefähr halbiert werden.

Für die kontinuierliche Betriebsweise sind mehrere Ausgestaltungen des Verfahrens möglich. Kontinuierlich durchflossene Rührbehälter sind als mindestens zweistufige Kaskade mit einer mittleren Verwellzeit von 10 bis 60 Minuten auszubilden. Bei einer anderen Variante der kontinuierlichen Betriebsweise werden vorgeheizte Fettsäure und Wasserstoffperoxid einem statischen Mischer zugeführt. Die Nachreaktion erfolgt in einer dem Mischer nachgeschalteten beheizten Rohrschlange.

Für alle Betriebsarten wird gewährleistet, daß keine Peroxide in die nächste Verarbeitungsstufe gelangen. Letzteres wird mittels Kaliumjodid bzw. Peroxid-Prüf-Stäbchen nachgewiesen; beim Chargenbetrieb nach jedem abgeschlossenen Ansatz und bei kontinuierlicher Fahrweise in regelmäßigen Abständen.
Die anschließende fraktionierte und/oder Geradeausdestillation der Fettsäure erfolgt in bekannter Weise. Aus der Umsetzung der Chromophore mittels H₂O₂ zu höhermolekularen Verbindungen resultiert naturgemäß eine größere Menge an Destillationsrückstand als bei der Destillation unbehandelter Fettsäuren. Die Zunahme der Rückstandsmenge hängt ab von der Menge der eingesetzten 30%igen H₂O₂-Lösung und ist bei Oleinen deutlich größer als bei gesättigten Fettsäuren.
Folgende Beziehungen wurden empirisch ermittelt:

| | |
|---|---|
| gesättigte Fettsäuren | M₂ = M₁ + n % H₂O₂ (30%ig) x 0,75 |
| Oleine | M₂ = M₁ + n % H₂O₂ (30%ig) x 2,5, |
| wobei | |
| | M₁ = Rückstandsmenge in % ohne H₂O₂-Behandlung und |
| | M₂ = Rückstandsmenge in % mit H₂O₂-Behandlung |

bedeuten.

Die Farbwerte der Destillate wurden vor und nach Thermotest mit einem handelsüblichen Gerät gemessen. Der Thermotest wurde wie folgt durchgeführt:
40 g Substanz, Glasreaktlonsgefäß 220 ml NS 29/32 mit aufgesetztem Rückflußkühler, Heizblock 200 ± 0,5 °C, 2h.
Im Durchschnitt wurden nach H₂O₂-Behandlung der Fettsäuren unter den zuvor erläuterten Bedingungen folgende Farbwerte der Destillate festgestellt:

| Fettsäure | vor Thermotest | nach Thermotest | |
|---|---|---|---|
| | | Lov. 5 1/4 " | |
| | APHA | Total 10xR+G | APHA |
| C₈/C₁₀-Fettsäure nach frakt. Abtrennung C₆ und Geradeausdestillation | 15 bis 25 | ca. 5 | 60 bis 80 |
| Stearine, dest. aus gehärteter Talgfettsäure | 20 bis 50 | ca. 10 | 80 bis 100 |
| Oleine pflanzlicher oder tierischer Herkunft | 80 bis 150 | 20 bis 50 | 250 bis 300 |

Die Farbwerte unter gleichen Bedingungen destillierter Fettsäuren und aus gleicher Rohfettsäure ohne vorherige Behandlung mit Wasserstoffperoxid waren deutlich höher. Die H₂O₂-Behandiung der Fettsäuren vor deren Destillation bewirkt außer der Verbesserung der Farbwerte vor und nach dem Thermotest eine Verringerung der Mengensumme der im Gaschromatogramm sichtbaren Nicht-Fettsäure-Komponenten und bei gesättigten Fettsäuen eine Absenkung der Rest-Jodzahl in den Fettsäuredestillaten. Als Nicht-Fettsäuren sind alle Komponenten zu verstehen, deren GC-peaks sich nicht eindeutig gesättigten oder ungesättigten monofunktionellen n-Carbonsäuren (d.h. Fettsäuren) zuordnen lassen.
Ähnliche Verhältnisse wurden auch bei den aus den Fettsäuren und nach üblichen Verfahren hergestellten Estern festgestellt.
Während die Farbunterschiede bei destillierten Estern zwar deutlich, aber relativ geringer sind als bei den zur Veresterung eingesetzten Fettsäuren mit bzw. ohne H₂O₂-Behandlung, sind die Qualitätsdifferenzen hinsichtlich Farbe und Thermostabilität bei nicht destillationsfähigen Estern gravierend.
Durch die vorgeschlagene Verfahrensweise werden die Nachteile der im Stand der Technik genannten Verfahren hinsichtlich deren Wirtschaftlichkeit, Umweltbelastung und möglicher Kontaminierung der Fettsäuren mit Fremdstoffen beseitigt. Die Verwendung der erfindungsgemäß hergestellten Fettsäuren zur Herstellung von Fettsäureestern führt im Vergleich zu unbehandelten Fettsäuren zu Produkten mit einer deutlich helleren Farbe und einer wesentlich verbesserten thermischen Farbstabilität.

### Beispiel 1

In einem 1I-Rundkolben, ausgestattet mit Rührer, Stickstoffeinleitung und Wasserabscheider werden 500 g der C₆/C₁₀-Fraktion aus Palmkernölfettsäure vorgelegt und auf 170 °C erhitzt. Unter Rühren werden 1 I/h Stickstoff ständig und 5 g H₂O₂ (30%ig) (1 % bezogen auf Fettsäuremenge) während 5 Minuten über Tauchrohre eingeleitet. Das Gasgemisch, bestehend aus Wasserdampf und Stickstoff, wird zur Kondensation des Wasserdampfes gekühlt und über einen belüfteten Wasserabscheider geführt. Nach beendeter H₂O₂-Zugabe wird bei 170 °C 30 Minuten nachgerührt, wobei bereits nach 10 Minuten keine Peroxide mehr nachwelsbar waren (mittels Kaliumjodid). Die so behandelte Fettsäure wird anschließend zur Abtrennung der C₆-Fettsäure bei einem Druck von 10 mbar fraktioniert und die C₈-Fraktion angeschnitten (Kopftemperatur bis 125 °C). Für die Fraktionierung wurde eine Oldershaw-Kolonne mit 20 Slebböden verwendet. Nach Abtrennung der C₆-Fraktion wird der Rückstand bei einem Druck von 10 mbar geradeaus destilliert. Ergebnisse hinsichtlich Rückstandsmenge sowie Farbe der destillierten C₈/C₁₀-Fettsäurefraktion vor und nach Thermotest sind in der abschließenden Tabelle angegeben.
Letztgenannte Fraktion wird mit Glycerin (polyol grade) in bekannter Weise verestert und Im Dünnschichtverdampfer bei 0,1 mbar destilliert. Farbe und Thermostabilität des hergestellten Capryl-/Caprinsäure-Triglycerids sind in der abschließenden Tabelle angegeben.

### Beispiel 2

In einem 1l-Rundkolben einer Rotavapor-Apparatur werden 500 g der C₆/C₁₀-Fraktion von Kokosspaltfettsäure vorgelegt. Das Ölbad ist auf eine Temperatur von 175 °C eingestellt. Der Kolben wird mit ca. 150 U/min bewegt und die Apparatur unter Normaldruck betrieben. Über Tauchrohre werden 1 l/h Stickstoff ständig und 15 g H₂O₂ (30%ig) (3 % bezogen auf Fettsäuremenge) während 15 Minuten über Tauchrohre eingeleitet. 25 Minuten nach Beendigung der Wasserstoffzugabe sind Peroxide mittels Prüf-Stäbchen nicht mehr nachweisbar, und nach weiteren 10 Minuten wird die Apparatur abgestellt.

Die Fettsäure wird analog Beispiel 1 destillativ aufgearbeitet, von der C₈/C₁₀-Fettsäure die Kennzahlen bestimmt und daraus unter den gleichen Bedingungen wie Im Beispiel 1 Capryl-/Caprinsäure-Triglycerid hergestellt. Die erzielten Ergebnisse sind in der abschließenden Tabelle angegeben.

### Beispiel 3

Die C₆/C₁₀-Fraktion der bei der destillativen Entsäuerung von Kokosöl gewonnenen Fettsäure wird mit einer Dosiergeschwindigkeit von 12 l/h durch einen Doppelrohrvorheizer gepumpt und dabei auf eine Temperatur von 205 °C gebracht. Parallel dazu werden 300 ml/h H₂O₂ 30 %lg, ca. 3 % bezogen auf die Fettsäuremenge, gefördert. Beide Dosierströme werden einem statischen Mischer (Standardmischer SMV/Fa. Sulzer) zugeführt. Die Mischung durchläuft anschließend eine Rohrschlange (Durchmesser 10 mm; Länge 38 m; Volumen ca. 31), deren Temperatur mittels Wärmeträgerheizbad auf ebenfalls 205 °C gehalten wird. Die Peroxidprüfung aller im Abstand von 30 Minuten gezogenen Proben am Schlangenaustritt war negativ. Unmittelbar hinter der beheizten Rohrschlange wird das Reaktlonsprodukt auf 70 °C abgekühlt und die Fettsäure analog Beispiel 1 destillativ aufgearbeitet. Von der erhaltenen C₈/C₁₀-Fettsäure werden die Kennzahlen bestimmt und daraus unter den gleichen Bedingungen wie im Beispiel 1 Capryl-/Caprinsäuretriglyzerid hergestellt. Die erzielten Ergebnisse sind in der abschließenden Tabelle angegeben.

### Beispiel 4

Als Rohfettsäure wird ein Olein eingesetzt, das aus einem Stearin-Olein-Trennprozeß von Talgfettsäure stammt. Dieses Olein wird mittels Vorheizer auf eine Temperatur von 170 °C erhitzt und mit einer Dosiergeschwindigkeit von 170 kg/h = ca. 200 l/h in eine zweistufige Rührwerkkaskade gefördert. Die beiden Rührwerksbehälter haben ein Nennvolumen von je 150 I und sind mit jeweils 100 I gefüllt. In die erste Stufe der Kaskade werden über ein Tauchrohr 1,7 kg/h ≅ ca. 1,9 l/h 30%iges H₂O₂ (1 % bezogen auf Fettsäuremenge) dosiert und über Tauchrohre in jeden der beiden Rührbehälter je 100 l/h Reinststickstoff eingeleitet. Beide Rührbehälter sind beheizbar, so daß die Produkttemperatur auf 165 bis 170 °C gehalten wird. Die Dämpfe beider Behälter werden über einen gemeinsamen Kühler geführt und die Kondensate in einer belüfteten Destillationsvorlage gesammelt. Bei allen im Abstand von 30 Minuten am Ablauf der 2. Stufe der Reaktorkaskade gezogenen Proben ist die Prüfung auf Peroxide negativ. Anschließend erfolgen bei 20 mbar Entgasung, Entsäuerung und 0,5 % Vorlaufabtrennung sowie die Destillation des Oleins bei 3 mbar mittels Fallfilmverdampfer.
Farbzahlen des destillierten Oleins und Menge Destillationsrückstand sind In der abschließenden Tabelle angegeben. Das auf diese Weise gewonnene Olein wird mit Decylalkohol (OHZ = 354,4) in Gegenwart von Zinn (II)-Oxalat verestert, das Reaktionsgemisch anschließend mit 1 % Bleicherde behandelt, filtriert und destillativ aufgearbeitet. Farbzahlen von Decyloleat vor und nach Thermotest sind in der abschließenden Tabelle angegeben.
Das gleiche Olein wird mit Oleylalkohol (JZ = 86,0; OHZ = 213,0) in Gegenwart von Zinn(II)-Oxalat verestert (Mol-verh. Fettsäure/Fettalkohol = 1:1,05), mit 1 % Bleicherde behandelt und filtriert. Farbzahlen des undestillierten Oleyloleates vor und nach Thermotest sind in der abschließenden Tabelle angegeben.

### Beispiel 5

500 g rohe Stearlnsäure aus der Härtung von Talgfettsäure (JZ = 0,8) werden mit 0,8 % einer 30%igen Wasserstoffperoxidlösung in der gleichen Apparatur und unter gleichen Bedingungen wie im Beispiel 1 beschrieben behandelt. Anschließend erfolgt die Geradeausdestillation des Stearins. im Destillat werden vor und nach Thermotest die Farbzahlen bestimmt, die in der abschließenden Tabelle angegeben sind.
Die auf diese Weise destillierte Stearlnsäure wird in Gegenwart von Zinn(II)-Oxid als Katalysator mit Cetyl-/Stearyl-Alkohol (OHZ = 213,6) im Molverhältnis 1:1,03 verestert, das Reaktionsgemisch anschließend mit 1 % Bleicherde behandelt, filtriert und vom Filtrat vor und nach Thermotest die Farbwerte ermittelt, die in der abschließenden Tabelle angegeben sind.

### Vergleichsbeispiele 1 bis 5

Analog wie in den Beispielen 1 bis 5 werden aus den gleichen Rohstoffen Fettsäuren und Fettsäureester hergestellt, lediglich mit dem Unterschied, daß die Rohfettsäuren nicht mit H₂O₂ behandelt werden. An den hergestellten Fettsäuren und Fettsäureestern werden In analoger Weise wie In den Beispielen 1 bis 5 die Eigenschaftswerte bestimmt. Die erzielten Ergebnisse sind in der nachfolgenden Tabelle angegeben.

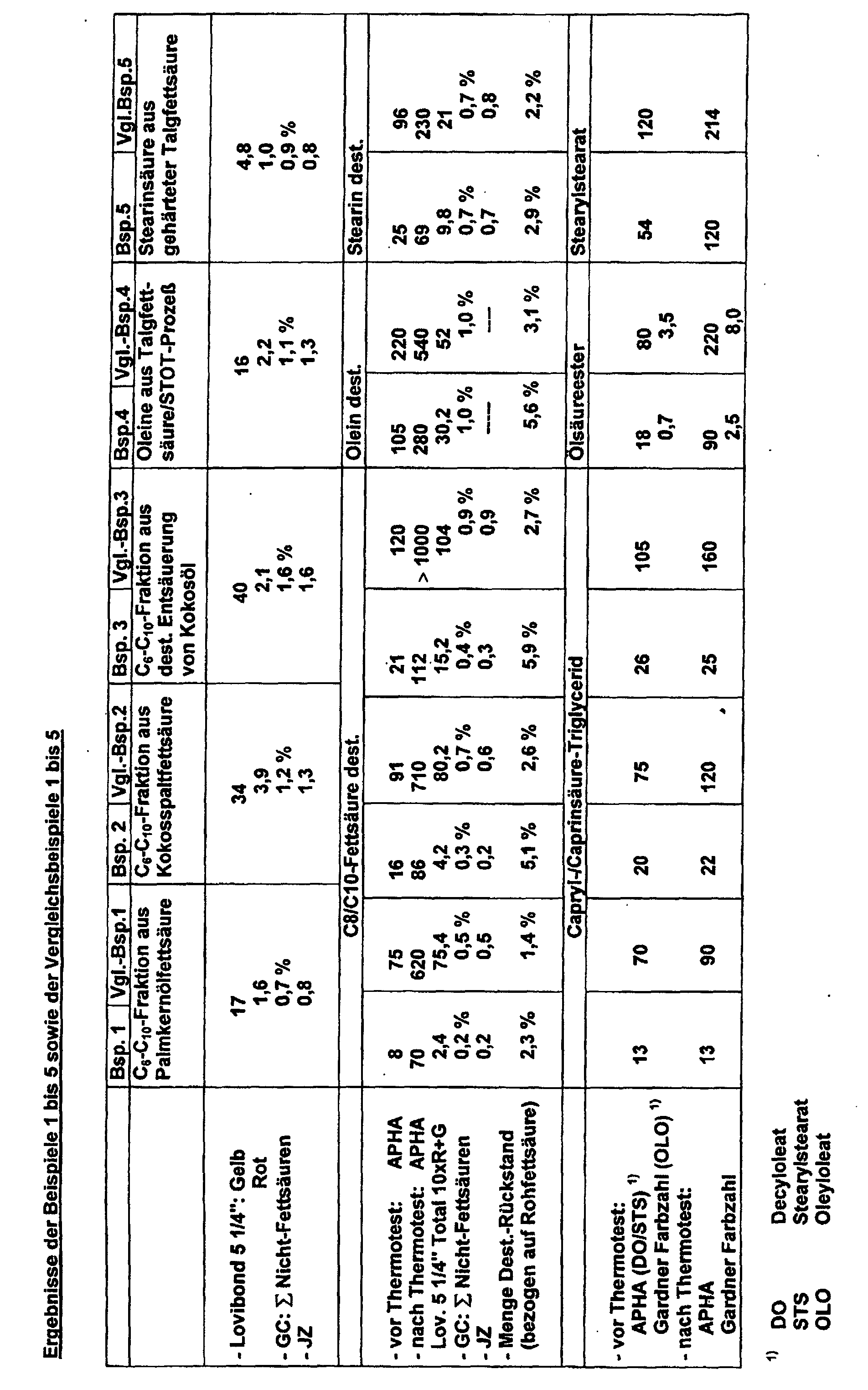

## Patentansprüche

1. Verfahren zur Behandlung natürlicher Fettsäuren vor deren anschließender Destillation durch Erhitzen der Rohfettsäure und Einleitung von Wasserstoffperoxid unter Vermischen, **dadurch gekennzeichnet, daß** die Rohfettsäure auf eine Temperatur von 150° C bis 230° C erhitzt wird, Wasserstoffperoxid als 3 bis 50 %ige Lösung in einer Menge von 0,5 bis 5 %, bezogen auf die zu behandelnde Rohfettsäuremenge, eingeleitet wird und nach Beendigung der Wasserstoffperoxideinleitung während mindestens 10 min bei gleicher Temperatur die Nachreaktion stattfindet, wobei die in den Rohfettsäuren enthaltenen Chromophore zu höhermolekularen Stoffen umgesetzt werden, die bei der anschließenden Destillation im Rückstand verbleiben.

2. Verfahren nach Anspruch 1, **dadurch gekennzeichnet, daß** während der gesamten Reaktionsdauer Stickstoff in einer definierten Menge eingeleitet wird.

3. Verfahren nach einem der Ansprüche 1 oder 2, **dadurch gekennzeichnet, daß** Wasserstoffperoxid als 30%ige Lösung in einer Menge von 1 bis 3 %, bezogen auf die zu behandelnde Rohfettsäuremenge, eingesetzt wird.

4. Verfahren nach einem der Ansprüche 1 bis 3, **dadurch gekennzeichnet, daß** die Rohfettsäure auf eine Temperatur von 165 bis 175° C erhitzt wird.

5. Verfahren nach einem der Ansprüche 1 bis 4, **dadurch gekennzeichnet, daß** Stickstoff in einer Menge von 1 bis 2 l/h x kg Rohfettsäure eingeleitet wird.

6. Verfahren nach einem der Ansprüche 1 bis 5, **dadurch gekennzeichnet, daß** die gesamte Reaktionszeit einschließlich Nachreaktionszeit 15 bis 60 min beträgt.

7. Verfahren nach einem der Ansprüche 1 bis 6, **dadurch gekennzeichnet, daß** die Behandlung diskontinuierlich durchgeführt wird, wobei die Wasserstoffperoxidlösung mit einer Geschwindigkeit von 0,1 bis 0,3 % (bezogen auf die Rohfettsäuremenge)/min, eingeleitet wird und nach Beendigung der Wasserstoffperoxideinleitung eine Nachreaktionszeit von 10 bis 30 min eingehalten wird.

8. Verfahren nach Anspruch 7, **dadurch gekennzeichnet, daß** die Wasserstoffperoxidlösung mit einer Geschwindigkeit von 0,2 %/min eingeleitet wird.

9. Verfahren nach einem der Ansprüche 7 oder 8, **dadurch gekennzeichnet, daß** die Nachreaktionszeit insgesamt 30 min beträgt.

10. Verfahren nach einem der Ansprüche 1 bis 6, **dadurch gekennzeichnet, daß** die Behandlung kontinuierlich in einer mindestens 2-stufigen Reaktorkaskade durchgeführt wird, wobei die Wasserstoffperoxideinleitung in der 1. Stufe und die Stickstoffbegasung in allen Stufen erfolgt und die Verweilzeit der Rohfettsäure insgesamt mindestens 60 min beträgt.

11. Verfahren nach einem der Ansprüche 1 bis 10, **dadurch gekennzeichnet, daß** die Rohfettsäure auf eine Temperatur von 200 bis 230 °C erhitzt wird und dadurch die notwendige Verweilzeit bei der Nachreaktion bis zum vollständigen Abbau der Peroxide auf 5 bis 20 Minuten in Abhängigkeit von der zugegebenen Wasserstoffperoxidmenge reduziert wird.

12. Verfahren nach einem der Ansprüche 1 bis 11, **dadurch gekennzeichnet, daß** bei kontinuierlicher H₂O₂-Behandlung die Rohfettsäure und Wasserstoffperoxid in einem statischen Mischer vermischt werden.

13. Verfahren nach einem der Ansprüche 1 bis 12, **dadurch gekennzeichnet, daß** bei kontinuierlicher H₂O₂-Behandlung die Nachreaktion in einer beheizten Rohrschlange erfolgt.

14. Verfahren nach einem der Ansprüche 1 bis 13, **dadurch gekennzeichnet, daß** als Rohfettsäure die C₆/C₁₀-Fraktionen von Kokos- oder Palmkernölfettsäure, Oleine pflanzlicher oder tierischer Herkunft sowie Stearine aus gehärteter Talgfettsäure eingesetzt werden.

## Claims

1. Process for treating natural fatty acids before their subsequent distillation by heating the crude fatty acid and introducing hydrogen peroxide with mixing, **characterized in that** the crude fatty acid is heated to a temperature of 150°C to 230°C, hydrogen peroxide is introduced as 3 to 50% strength solution in an amount of 0.5 to 5%, based on the amount of crude fatty acid to be treated, and after completion of hydrogen peroxide introduction the secondary reaction takes place at the same temperature for at least 10 min, the chromophores present in the crude fatty acids being converted to higher-molecular-weight substances which remain in the residue in the subsequent distillation.

2. Process according to Claim 1, **characterized in that** nitrogen is introduced at a defined rate during the entire reaction period.

3. Process according to one of Claims 1 or 2, **characterized in that** hydrogen peroxide is used as a 30% strength solution in an amount of 1 to 3%, based on the amount of crude fatty acid to be treated.

4. Process according to one of Claims 1 to 3, **characterized in that** the crude fatty acid is heated to a temperature of 165 to 175°C.

5. Process according to one of Claims 1 to 4, **characterized in that** nitrogen is introduced at a rate of 1 to 2 l/h × kg of crude fatty acid.

6. Process according to one of Claims 1 to 5, **characterized in that** the entire reaction time, including secondary reaction time, is 15 to 60 min.

7. Process according to one of Claims 1 to 6, **characterized in that** the treatment is carried out batchwise, the hydrogen peroxide solution being introduced at a rate of 0.1 to 0.3% (based on the amount of crude fatty acid)/min, and after completion of the hydrogen peroxide introduction a secondary reaction time of 10 to 30 min is maintained.

8. Process according to Claim 7, **characterized in that** the hydrogen peroxide solution is introduced at a rate of 0.2%/min.

9. Process according to one of Claims 7 or 8, **characterized in that** the secondary reaction time is in total 30 min.

10. Process according to one of Claims 1 to 6, **characterized in that** the treatment is carried out continuously in an at least 2-stage reactor cascade, the hydrogen peroxide being introduced in the first stage and the nitrogen gas being passed through in all stages and the residence time of the crude fatty acid overall being at least 60 min.

11. Process according to one of Claims 1 to 10, **characterized in that** the crude fatty acid is heated to a temperature of 200 to 230°C and as a result the required residence time in the secondary reaction up to complete breakdown of the peroxides is reduced to 5 to 20 minutes, depending on the amount of hydrogen peroxide added.

12. Process according to one of Claims 1 to 11, **characterized in that**, in the case of continuous H₂O₂ treatment, the crude fatty acid and hydrogen peroxide are mixed in a static mixer.

13. Process according to one of Claims 1 to 12, **characterized in that**, in the case of continuous H₂O₂ treatment, the secondary reaction takes place in a heated pipe coil.

14. Process according to one of Claims 1 to 13, **characterized in that** the crude fatty acid used is the C₆/C₁₀ fractions of coconut oil fatty acid or palm kernel oil fatty acid, oleins of vegetable or animal origin, and stearins from hardened tallow fatty acid.

## Revendications

1. Procédé pour le traitement d'acides gras naturels avant leur distillation subséquente, par chauffage de l'acide gras brut et introduction de peroxyde d'hydrogène avec mélange, **caractérisé en ce que** l'acide gras brut est chauffé à une température de 150°C à 230°C, du peroxyde d'hydrogène est introduit sous forme de solution à 3-50 %, en une quantité de 0,5 à 5 %, par rapport à la quantité d'acide gras brut à traiter, et une fois terminée l'introduction de peroxyde d'hydrogène, la post-réaction a lieu pendant au moins 10 minutes à la même température, ce par quoi les chromophores contenus dans les acides gras bruts sont convertis en substances de masse moléculaire élevée qui restent dans le résidu lors de la distillation subséquente.

2. Procédé selon la revendication 1, **caractérisé en ce que** pendant toute la durée de la réaction on fait passer de l'azote en une quantité définie.

3. Procédé selon la revendication 1 ou 2, **caractérisé en ce que** le peroxyde d'hydrogène est utilisé sous forme de solution à 30 %, en une quantité de 1 à 3 %, par rapport à la quantité d'acide gras brut à traiter.

4. Procédé selon l'une quelconque des revendications 1 à 3, **caractérisé en ce que** l'acide gras brut est chauffé à une température de 165 à 175°C.

5. Procédé selon l'une quelconque des revendications 1 à 4, **caractérisé en ce qu'**on fait passer de l'azote en une quantité de 1 à 2 l/h × kg d'acide gras brut.

6. Procédé selon l'une quelconque des revendications 1 à 5, **caractérisé en ce que** la durée totale de la réaction, y compris le temps de post-réaction, va de 15 à 60 minutes.

7. Procédé selon l'une quelconque des revendications 1 à 6, **caractérisé en ce que** le traitement est effectué en mode discontinu, la solution de peroxyde d'hydrogène étant introduite à une vitesse de 0,1 à 0,3 % (par rapport à la quantité d'acide gras brut)/minute, et, une fois terminée l'introduction de peroxyde d'hydrogène, un temps de post-réaction de 10 à 30 minutes étant respecté.

8. Procédé selon la revendication 7, **caractérisé en ce que** la solution de peroxyde d'hydrogène est introduite à une vitesse de 0,2 %/minute.

9. Procédé selon la revendication 7 ou 8, **caractérisé en ce que** le temps de post-réaction est au total de 30 minutes.

10. Procédé selon l'une quelconque des revendications 1 à 6, **caractérisé en ce que** le traitement est effectué en continu dans une cascade de réacteurs d'au moins 2 étages, l'introduction de peroxyde d'hydrogène étant effectuée dans le 1^{er} étage et l'insufflation d'azote étant effectuée dans tous les étages, et le temps de séjour de l'acide gras brut étant au total d'au moins 60 minutes.

11. Procédé selon l'une quelconque des revendications 1 à 10, **caractérisé en ce que** l'acide gras brut est chauffé à une température de 200 à 230°C, et le temps de séjour requis lors de la post-réaction jusqu'à la dégradation totale des peroxydes est ainsi réduit à 5-20 minutes en fonction de la quantité ajoutée de peroxyde d'hydrogène.

12. Procédé selon l'une quelconque des revendications 1 à 11, **caractérisé en ce que** lors du traitement continu par H₂O₂, l'acide gras brut et le peroxyde d'hydrogène sont mélangés dans un mélangeur statique.

13. Procédé selon l'une quelconque des revendications 1 à 12, **caractérisé en ce que** lors du traitement continu par H₂O₂, la post-réaction s'effectue dans un serpentin chauffé.

14. Procédé selon l'une quelconque des revendications 1 à 13, **caractérisé en ce qu'**on utilise comme acide gras brut les fractions en C₆/C₁₀ d'acide gras de coco ou d'acide gras de palmiste, des oléines d'origine animale ou végétale, ainsi que des stéarines provenant d'acide gras de suif hydrogéné.
